# DEMANDE DE BREVET EUROPEEN

(11) **EP 1 588 685 A1**
(43) Date de publication de la demande: **26.10.2005**
(21) Numéro de dépôt: 05356067.8
(22) Date de dépôt: 19.04.2005
(51) Int. Cl.: A61H 33/00

(54) **Système de balnéothérapie muni d'une carte à puce permettant de personnaliser les massages**

(30) Priorité: 20.04.2004 FR 0404148
(71) Demandeur: Prestige Sanitaire, 04100 Manosque (FR)
(72) Inventeur: Pertosa, Yves, 69310 Pierre-Bénite (FR)

(57) **Abrégé**

L'invention concerne un dispositif permettant de personnaliser les massages de balnéothérapie grâce à une carte à puce nominative et personnaliser en ce qu'il prend en compte la morphologie et les besoins de chaque utilisateur. L'invention comprend la mise en oeuvre des différentes phases pour arriver aux résultats.

L'invention est constituée d'un lecteur de carte à puce ( 4 ) étanche placer sous ou sur la baignoire qui transmet l'analyse des données du questionnaire enregistrées sur la carte à puce ( 1 ) au boîtier électronique ( 2 ) qui commande les différents composants de la balnéothérapie (3)

## Description

La présente invention concerne un dispositif pour personnaliser les massages de balnéothérapie grâce à un programme qui fait une analyse des données d'un questionnaire et qui transmet par la suite son analyse au programme qui orchestre les différents composants de la baignoire.

La baignoire équipée de système de massage comprenant des buses incorporées ou des systèmes de matelas amovibles reliés à des pompes ou des turbines à air, est utilisé depuis longtemps et la société Prestige Sanitaire a constaté que la plupart du temps l'ergonomie de la baignoire est mal adaptée à la morphologie de la personne. De plus, les buses sont mal positionnées. Ainsi le massage est inadapté car chaque utilisateur est différent.

Le dispositif selon l'invention permet de personnaliser le massage et de le rendre donc adapté à chaque personne.
La société Prestige Sanitaire a donc imaginé :

1 questionnaire individualisé pour chaque utilisateur désirant personnaliser les séances de balnéothérapie.

1 logiciel d'analyse des données qui calcule l'incidence des réponses aux questionnaires sur les phases de fonctionnement de la balnéothérapie.

Par la suite ces données sont compilées et expédiées vers le logiciel de programmation de la carte à puce.

Ces données seront rassemblées dans une carte à puce (1) qui sera éditée. Cette carte à puce transmettra ces données au lecteur de carte à puce ( 4 ) étanche intégré sous ou sur la coque de la baignoire. Le lecteur de carte à puces transmettra alors les données au boîtier électronique ( 2 ) qui pilotera les différents composants de la baignoire ( 3 ) (moteurs, turbines, électrovannes, jets, pompes ) Chaque utilisateur à sa propre carte avec son programme de balnéothérapie. Un simple passage de la carte sur le bord de la baignoire enclenchera la mise en marche du programme inscrit sur la carte à puce. Un autre passage de la carte à puce sur le bord de la baignoire permettra d'arrêter le programme et donc le fonctionnement de la baignoire.

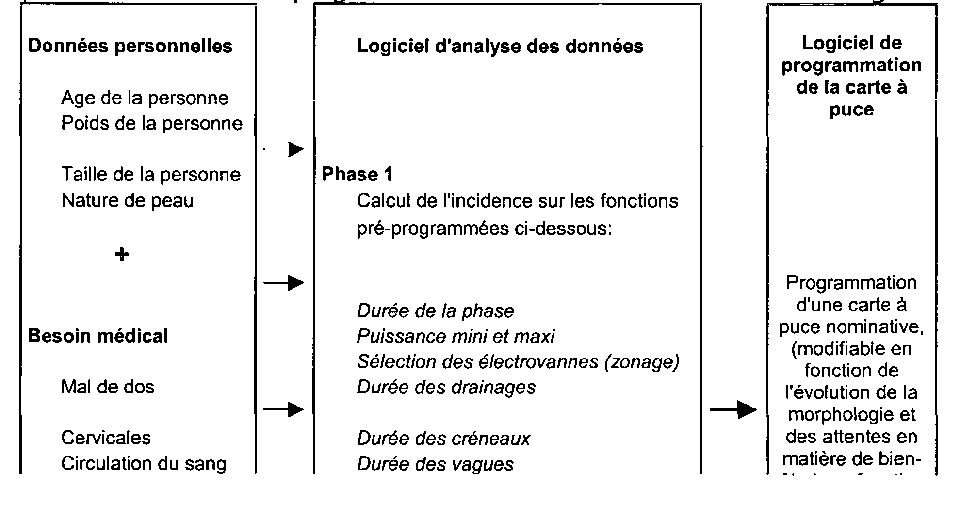

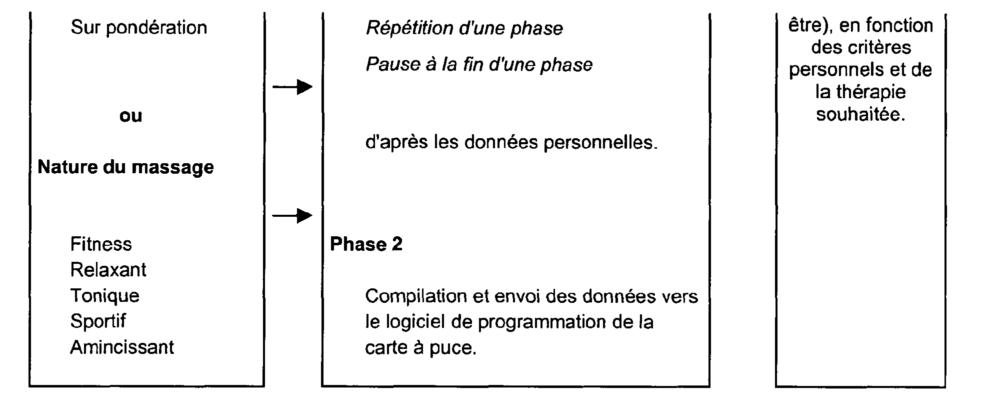

Le questionnaire se compose de données personnelles et de l'expression des besoins médicaux de l'utilisateur :

Voici un exemple de questionnaire que l'utilisateur doit remplir pour que son massage soit personnalisé :

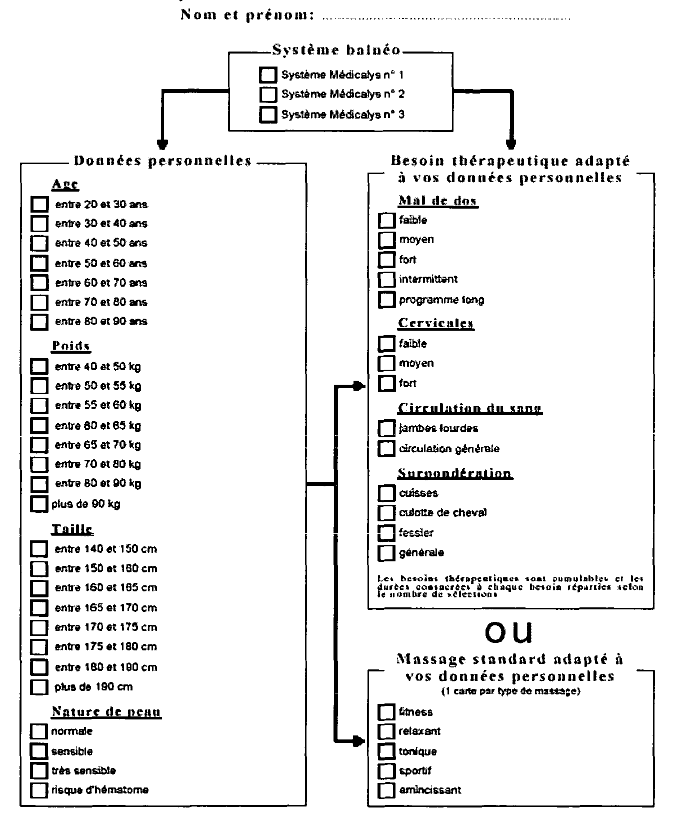

Le questionnaire sert à affiner chaque point de massage en fonction des réponses que l'utilisateur a inscrit dans le questionnaire.

Ce questionnaire se compose de données personnelles et de l'expression des besoins thérapeutiques de l'utilisateur :

Les données personnelles sont l'âge (échelle de 20 à 90 ans), le poids (échelle de 40 à 90 kilos), la taille (échelle de 140 à 190 centimètres), nature de peau (normale, sensible, très sensible, risque d'hématome).
Ces donnés personnelles comme l'âge de l'utilisateur reflètent que les besoins en terme de balnéothérapie sont différents d'un utilisateur à l'autre. Une personne âgée ne supportera pas la même durée de massage et la même puissance des moteurs qu'une jeune personne. De plus, il faudra augmenter le temps des pauses entre chaque phases. C'est pourquoi les moteurs varient d'une puissance de 1 à 100 afin de s'adapter à toutes les personnes y compris les types de peau.

Les besoins thérapeutiques s'expriment surtout par quatre critères qui sont le mal de dos (faible, moyen, fort intermittent, programme long), les cervicales (faible, moyen, fort), la circulation du sang (jambes lourdes, circulation générale), la sur pondération (cuisses, culotte de cheval, fessier, générale). Les besoins thérapeutiques sont cumulables et les durées consacrées à chaque besoin sont réparties selon le nombre de sélections.
Les besoins médicaux sont l'expression des zones prioritaires de massage. Si l'utilisateur à des problèmes de circulations du sang, le massage se fait des pieds jusqu'au coeur : les moteurs s'enclencheront de manière successive du bas de la baignoire vers le haut de la baignoire.

Les massages fitness, relaxant, tonique, sportif, amincissant sont modifiés en fonction des données personnelles enregistrées dans le questionnaire. L'utilisateur choisit l'un de ces programmes et ils sont modifiés en conséquences. Il y a une carte personnalisée par type de massage réajusté à l'utilisateur.
L'utilisateur rempliera soit la partie données personnelles et besoins médicaux, soit la partie données personnelles et massages standard.

Ce questionnaire est analysé par un programme qui calcule les incidences des réponses au questionnaire sur les phases de fonctionnement de la balnéothérapie.

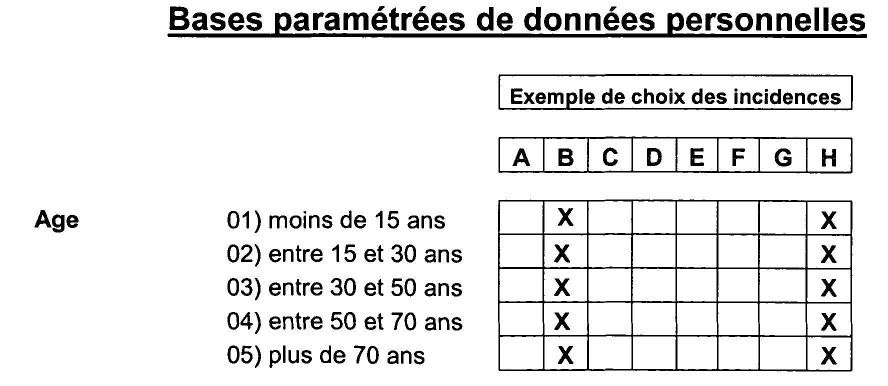

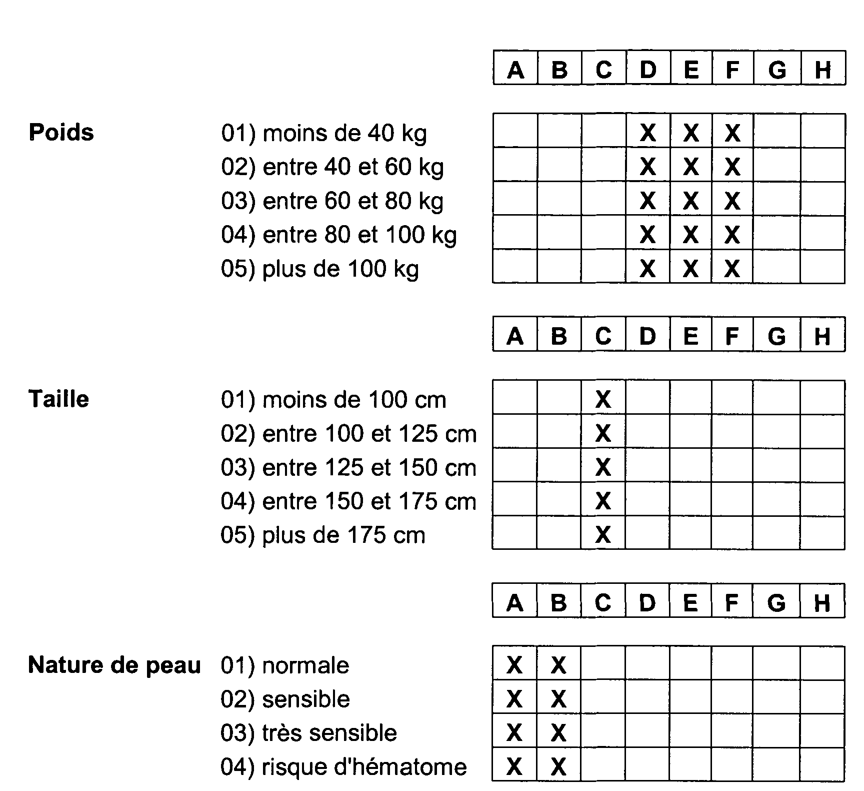

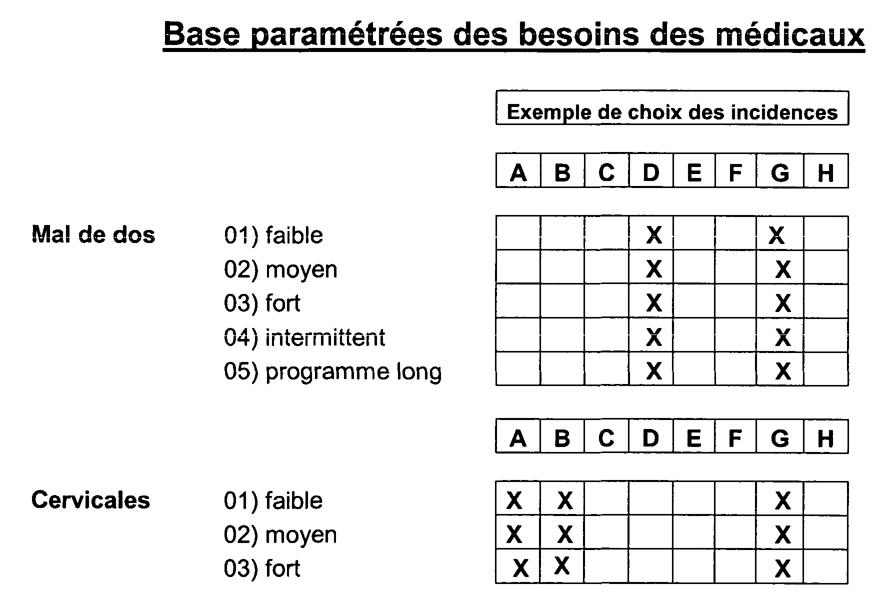

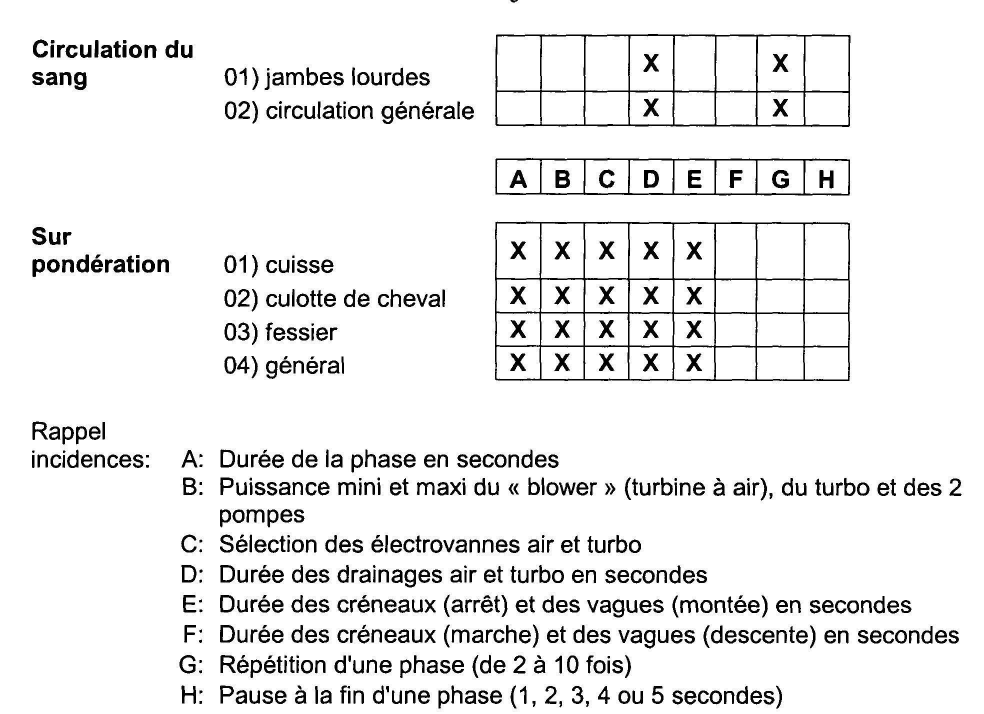

Le choix des incidences et des données personnelles sont modifiables et améliorables en adéquation avec les besoins sociétales, budgétaires et les perfectionnements des appareils de massages.

| **Programme modifiable selon données** **personnelles** | **Type** | **Opération de contrôle** |
|---|---|---|
| OUI | A | Durée de la phase en secondes |
| OUI | B | Puissance mini et maxi du « blower » (turbine à air), du turbo et des 2 pompes |
| NON | | Régime continu, créneaux, effet vague, rampe et alternatif (pompes uniquement) |
| OUI | C | Sélection des électrovannes air et turbo |
| OUI | D | Durée des drainages air et turbo en secondes |
| OUI | E | Durée des créneaux (arrêt) et des vagues (montée) en secondes |
| OUI | F | Durée des créneaux (marche) et des vagues (descente) en secondes |
| OUI | G | Répétition d'une phase (de 2 à 10 fois) |
| OUI | H | Pause à la fin d'une phase (1, 2, 3, 4 ou 5 secondes) |
| NON | | Chromothérapie |

Ainsi, en fonction de l'âge de l'utilisateur, la durée de pause entre chaque phase variera. Si l'utilisateur a entre 30 et 40 ans son corps s'habitue plus vite aux changements de phases dans le massage. Par exemple, le passage entre une phase Electrovanne air (jambes) et Electrovanne air (fessier) pourra être plus court de deux secondes, au lieu de cinq secondes pour un utilisateur plus vieux. Plus le temps de pause entre chaque phase est long, plus l'utilisateur a la possibilité de s'adapter. Une personne âgée aura donc moins un sentiment d'épuisement.
Si l'utilisateur pèse entre 80 et 90 Kilos, le processus de massage est anémié par la masse graisseuse donc il est nécessaire d'augmenter le temps entre chaque phase pour atteindre le système nerveux, veineux et musculaire et éliminer la graisse.
Si l'utilisateur mesure entre 150 et 160 centimètres, les phases si elles le prévoient, baisseront la puissance de l'arriver d'eau ou actionnera l'électrovanne pour stopper cette arrivée afin que l'utilisateur ne se sente pas noyé par les remous de l'eau.
Si l'utilisateur à des risques d'hématomes les moteurs augmentent très progressivement leur puissance, ce qui augmentera le temps de la phase. Une fois que le moteur aura atteint sa phase maximum, il restera que très peu de temps sur le point de massage afin de ne pas provoquer d'hématomes.

L'analyse des incidences est transférée dans la puce d'une carte nominative. La carte à puce est programmée par un logiciel de programmation de carte à puce.

### Exemple de nomenclature pour la programmation

| **Désignation** | **Valeurs possibles** | **Unité** | **bit** | **Sur** **carte** **position** | **octet** | **Fichier texte position** |
|---|---|---|---|---|---|---|
| | | | | | | |
| **n° de phase** | 1 à 20 | | | | **2** | 1 à 2 |
| | | | | | | |
| **durée de la phase** | 1 à 63 | seconde | **6** | 1 à 6 | **2** | 3 à 4 |
| | | | | | | |
| **puissance « blower » (turbine à air) min** | 0 à 30 | % / 2 | **5** | 7 à 11 | **2** | 5 à 6 |
| **puissance « blower » (turbine à air) max** | 0 à 50 | % / 2 | **6** | 12 à 17 | **2** | 7 à 8 |
| **R E C** | 0, 1, 2 ou 3 | | **2** | 18à19 | **1** | 9 |
| **EVair1** | 0 ou 1 | | **1** | 20 | **1** | 10 |
| **EVair2** | 0 ou 1 | | **1** | 21 | **1** | 11 |
| **EVair3** | 0 ou 1 | | **1** | 22 | **1** | 12 |
| **drainage air** | 0 à 7 | seconde | **3** | 23 à 25 | **1** | 13 |
| **puissance du turbo min** | 0 à 30 | % / 2 | **5** | 26 à 30 | **2** | 14 à 15 |
| **puissance du turbo max** | 0 à 50 | %/2 | **6** | 31 à 36 | **3** | 16 à 18 |
| **R E C** | 0, 1, 2 ou 3 | | **2** | 37 à 38 | **1** | 19 |
| **Electrovanne turbo2** | 0 ou 1 | | **1** | 40 | **1** | 21 |
| **Electrovanne turbo3** | 0 ou 1 | | **1** | 41 | **1** | 22 |
| **Electrovanne turbo4** | 0 ou 1 | | **1** | 42 | **1** | 23 |
| **Electrovanne turbo5** | 0 ou 1 | | **1** | 43 | **1** | 24 |
| **drainage turbo** | 0 à 7 | seconde | **3** | 44 à 46 | **1** | 25 |
| **puissance pompe min** | 0 à 30 | % / 2 | **5** | 47 à 51 | **2** | 26 à 27 |
| **puissance pompe1 max** | 0 à 50 | % / 2 | **6** | 52 à 57 | **3** | 28 à 30 |
| **puissance pompe2 max** | 0 à 50 | %/2 | **6** | 58 à 63 | **3** | 31 à 33 |
| **Rampe Effet vague Crenaux** | 0, 1, 2, 3 ou 4 | | **3** | 64 à 66 | **1** | 34 |
| **Chromotérapie** | 0 à 15 | | **4** | 67 à 70 | **2** | 35 à 36 |
| **Créneau, vague (arrêt, /)** | 0 à 7 | seconde | **3** | 71 à 73 | **1** | 37 |
| **Créneau, vague (marche, \)** | 0 à 7 | seconde | **3** | 74 à 76 | **1** | 38 |
| **Répétition ou pause** | 0 à 15 | | **4** | 77 à 80 | **2** | 39 à 40 |
| **Longueur enregistrement (sans caractères de contrôle** | | | | 80 bits | | 40 octets |

| **Chromothérapie** | | **Répétition ou pause** | | | | |
|---|---|---|---|---|---|---|
| **0=** sans effet | | **1** = 1 fois la phase | | | | |
| **1** = couleur jaune | | **2**= 2 fois la phase | | | | |
| **2**= couleur verte | | **3**= 3 fois la phase | | | | |
| **3**= couleur bleue | | **4**= 4 fois la phase | | | | |
| **4**= couleur rouge | | **5**= 5 fois la phase | | | | |
| **5**= couleur jaune-vert | | **6**= 6 fois la phase | | | | |
| **6**= couleur jaune-rouge | | **7**= 7 fois la phase | | | | |
| **7**= couleur jaune-bleu | | **8**= 8 fois la phase | | | | |
| **8**= couleur vert-bleu | | **9**= 9 fois la phase | | | | |
| **9**= couleur vert-rouge | | **10**= 10 fois la phase | | | | |
| **10**= couleur bleu-rouge | | **11**= pause 1 secondes | | | | |
| **11**= défilement des couleurs (très lent) | | **12**= pause 2 secondes | | | | |
| **12**= défilement des couleurs (lent) | | **13**= pause 3 secondes | | | | |
| **13**= défilement des couleurs (moyen) | | **14**= pause 4 secondes | | | | |
| **14**= défilement des couleurs (rapide) | | **15**= pause 5 secondes | | | | |

| **Blower, turbo, pompes 1 et 2** | | | | | | |
|---|---|---|---|---|---|---|
| **0= sans effet** | | | | | | |
| **1= Effet vague**: variation min% à max% sur 5 sec puis max% vers min% | | | | | | |
| **2= Créneaux mini-maxi:** 5 sec à min% et 5 sec à max% | | | | | | |
| **3= Rampe**: variation lente de min% à max% sur la durée de la phase | | | | | | |
| **4= Alternatif:** 5 sec pompe1 à max% et 5 sec pompe2 à max% | | | | | | |

La programmation de la carte peut être modifiée en fonction de l'évolution de la morphologie, des besoins en matière de bien être ou de la thérapie souhaitée.

Ce système de balnéothérapie, muni d'une puce permettant de personnaliser les massages, peut-être appliqué à la baignoire « MEDICALYS » comprenant :
- 1 coque de 184x98 cm ergonomique en polyester percée à 29 endroits
- 1 ou plusieurs pompes hydro variables (30 m3/h)
- 1 turbojet 1100 watts
- 1 turbine à air 1100 Watts
- 1 réchauffeur 300 watts
- 1 sensor niveau manque d'eau
- 1 support anti-vibration
- 1 vidage et trop-plein monté
- 1 crépine d'aspiration de sécurité
- 14 hydrojets orientés à 45° (5) et (7) jets à propulsion air et /ou eau sont disposés à 45° (5) grâce à une forme particulière de la baignoire.
- 4 jets à propulsions air et /ou eau à 45° (7) placés entre les jambes de l'utilisateur.
- 10 hydrojets orientés à 90°(6)
- 1 hydrojet cervical
- 1 hydrojet fessier
- 2 buses tourbillonnantes pédiluves
- 14 injecteurs d'air lombaires
- 4 injecteurs d'air fessiers
- 10 injecteurs d'air jambes+cuisses
- Air venturi
- 1 ou plusieurs turbines à air variables
- 1 ou plusieurs électrovannes sur le circuit air du système massant
- 1 ou plusieurs électrovannes sur le circuit eau du système massant
- 1 électrovanne pour le remplissage de la baignoire
- 1 électrovanne pour la vidange
- 1 électrovanne pour le rinçage
- jets air/eau encastrés dans des gorges au fond de la baignoire afin qu'elle reste confortable même sans eau. Ces gorges permettent également d'effectuer un drainage d'air (9) sous le corps plus efficace que les jets saillants habituellement utilisés qui sont obstrués automatiquement par le corps des utilisateurs (8).
L'ensemble n'est pas limitatif. L'ensemble peut s'adapter sur une autre baignoire que celle-ci.
Le dispositif peut s'intégrer dans tout système de balnéothérapie ayant des composants similaires à ceux cités précédemment.
Ainsi, la coque peut avoir un à plusieurs pompe à eau, une à plusieurs turbines à air, plusieurs jets propulsant de l'air et/ou de l'eau...

Dans le massage de balnéothérapie, il y a un nombre de phases variable. Plus le système de balnéothérapie comporte des composants (moteurs, turbines, électrovannes...) plus il est possible de faire des phases variés dans le fonctionnement du système de balnéothérapie.

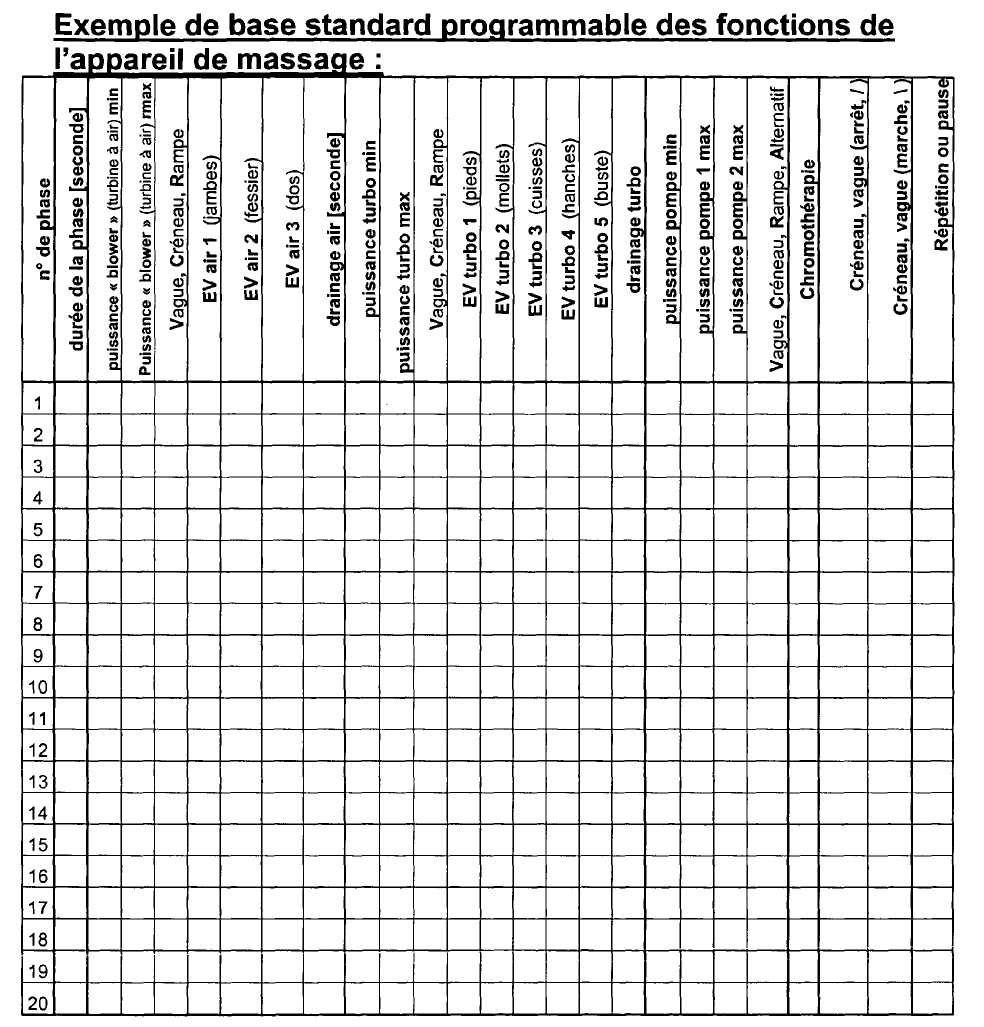

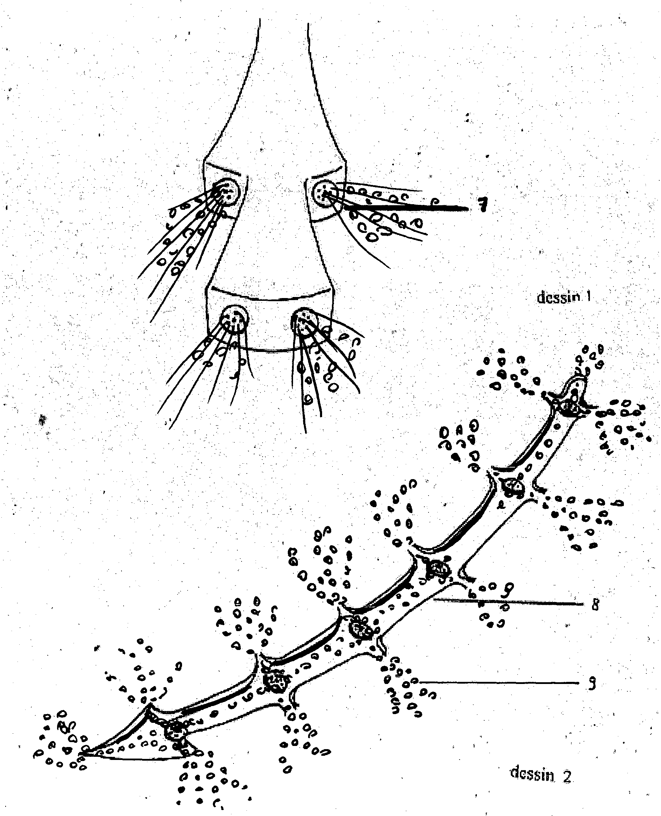

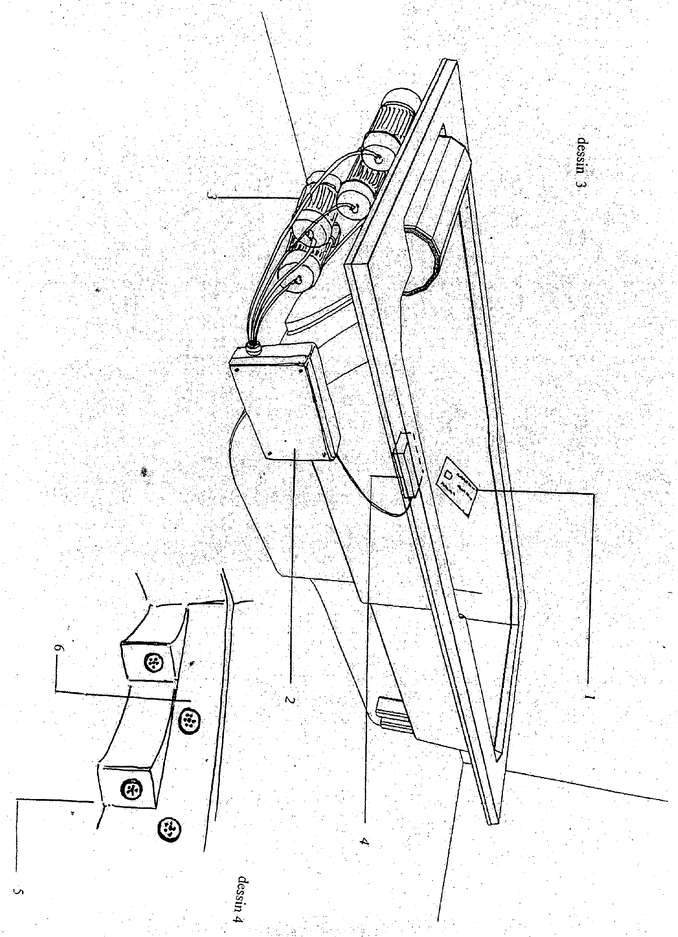

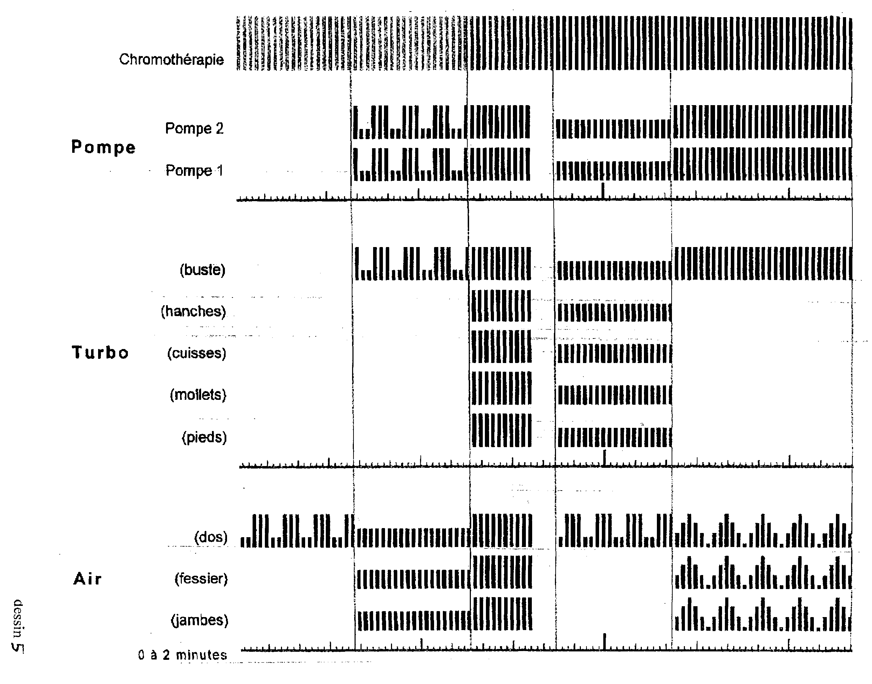

### Ainsi, nous pouvons exposer un exemple du massage : figure 5

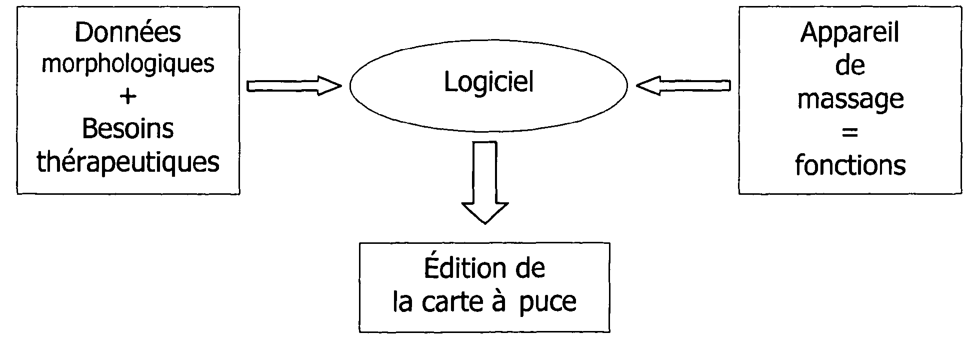

## Revendications

1. Dispositif pour permettre la personnalisation des massages de balnéothérapie **caractérisé en ce qu'**il comporte une baignoire avec un lecteur de carte puce ( 4 ) étanche et placé sur ou sous de la baignoire.

2. Dispositif selon la revendication 1 **caractérisé en ce que** le lecteur de carte à puce transmet les données enregistrées de la carte à puce (1) au boîtier électronique qui actionne les composants du système de balnéothérapie ( 3 ) en fonction de l'analyse effectuée par le logiciel.

3. Dispositif selon la revendication 2 **caractérisé en ce que** un simple passage de la carte à puce sur le bord de la baignoire enclenchera la mise en marche du programme inscrit sur la carte à puce. Un autre passage de la carte à puce sur le bord de la baignoire permettra d'arrêter le programme et donc le fonctionnement de la baignoire.

4. Dispositif selon la revendication 3 **caractérisé en ce que** des jets à propulsion air et /ou eau sont disposés à 45° ( 5 ) grâce à une forme particulière de la baignoire.

5. Dispositif selon la revendication 4 **caractérisé en ce que** des jets d'air/eau encastrés dans des gorges draineuses ( 8 ) sont répartis dans le fond de la baignoire.

6. Dispositif selon la revendication 5 **caractérisé en ce que** des jets à propulsions air et /ou eau à 45° ( 7 ) sont placés entre les jambes de l'utilisateur.

7. Procédé de fonctionnement du dispositif selon l'une quelconque des revendications 1 à 6 **caractérisé par** les étapes suivantes: Réponses au questionnaire, l'analyse des réponses de l'utilisateur au questionnaire par un logiciel qui déterminera les zones prioritaires de massage, l'édition de la carte à puce individuelle où figurera l'analyse de ces données, le passage de la carte à puce près du lecteur de carte à puce étanche, le déclenchement par le lecteur des différents composants de la baignoire suivant la détermination des zones prioritaires de massage.
